# EUROPEAN PATENT SPECIFICATION

(11) **EP 0 415 958 B1**
(45) Date of publication and mention of the grant of the patent: **21.05.1997**
(21) Application number: 89905267.4
(22) Date of filing: 30.01.1989
(51) Int. Cl.: C12N 15/00, C12N 1/20

(54) **A P1 BACTERIOPHAGE CLONING SYSTEM FOR DNA FRAGMENTS AS LARGE AS 95 KB**
P1-BAKTERIOPHAGEN-KLONIERUNGSSYSTEM FÜR BIS ZU 95 KB GROSSEN DNA-FRAGMENTEN
SYSTEME DE CLONAGE DU BACTERIOPHAGE P1 POUR FRAGMENTS D'ADN DONT LA TAILLE PEUT ATTEINDRE 95 KB

(30) Priority: 15.04.1988 US 182112
(43) Date of publication of application: 13.03.1991
(73) Proprietor: THE DU PONT MERCK PHARMACEUTICAL COMPANY, Wilmington, Delaware 19880-0025 (US)
(72) Inventor: STERNBERG, Nat, Leon, West Chester, PA 19382 (US)
(74) Representative: von Kreisler, Alek, Dipl.-Chem.
(86) International application number: US8900368
(87) International publication number: WO8909826

(56) References cited:
- EP-A- 183 571
- WO-A-91/02801
- JOURNAL OF MOLECULAR BIOLOGY, vol. 194, 05 Apriil 1987, Academic Press, New York, NY (US); N. STERNBERG et al., pp. 463-468; and N. STERNBERG et al., pp. 469-479
- JOURNAL OF MOLECULAR BIOLOGY, vol. 187, 20 January 1986, Academic Press, New York, NY (US); N. STERNBERG et al., pp. 197-212

## Description

### FIELD OF THE INVENTION

This invention relates to a bacteriophage P1 cloning system to control the amplification of DNA fragments as large as 95 kb in size.

### BACKGROUND OF THE INVENTION

Recombinant DNA technology has made it possible to clone and amplify DNA fragments from the chromosomes of high eucaryotes (plants and animals) using vectors that can be propagated in bacteria such as Escherichia coli (E. coli).

If DNA is to be introduced directly into bacteria that have been made competent for that uptake, then the DNA must be relatively small (less than 20 kb) since the efficiency of DNA uptake into cells decreases dramatically as the size of the DNA increase above 20 kb. Accordingly, alternate delivery routes have been sought in order to introduce large DNA into bacteria. The technique of packaging bacteriophage vector DNA into virus particles was developed to meet this need. It has the advantage of delivering the packaged DNA to cells by infection with near unit efficiency. Bruning et al., Gene 4, 85-107 (1978) and Collins et al., Proc. Natl. Acad. Sci., 75, 4242-4246 (1978) took advantage of the in vitro packaging reaction of bacteriophage lambda, developed by Sternberg et al., Gene 1, 255-280 (1975) to package large inserts cloned into cosmid vectors, vectors that are fusions of a plasmid and a bacteriophage lambda cos site. The cos site provides the recognition element needed to initiate the packaging of DNA into a lambda bacteriophage head.

The major disadvantage of the lambda - bacteriophage in vitro packaging reaction is that the lambda bacteriophage head cannot accommodate more than 49.5 kb of DNA. Thus, taking into account that the cos vector itself is about 2 kb in size, then no more than 47 kb of clonable DNA can be inserted into the vector and packaged into a lambda bacteriophage head. (Murray, Lambda II 236, 395-432, Cold Spring Harbor Laboratory, Cold Spring Harbor, New York 1986).

Accordingly, cloning genes bigger than 50 kb cannot be accomplished, and mapping chromosomal segments of DNA that are several megabases in size by "walking" or "jumping" protocols is difficult, given the limitations of the lambda bacteriophage system. Researchers have explored two systems as potential tools for cloning larger DNA fragments: phage systems with head sizes larger than bacteriophage lambda and yeast cloning vectors.

Rao et al., J. Mol. Biol. 185, 565-578 (1985), demonstrated that T4 DNA (about 165 kilobases) could be packaged into bacteriophage T4 heads in vitro. The efficiency of that reaction is 10⁴-10⁵ plaque-forming units (PFU) per microgram of added T4 DNA. However, Black, Gene 46, 97-101 (1986), showed that efforts to package vector DNA of various sorts into T4 heads and recover that DNA following injection into appropriate bacteria was no more efficient that 10²-10³ PFU per microgram of added DNA. Black's result suggests that it may be very difficult to use the T4 packaging system to generate a complete library of 150 kb inserts of a complex genome such as that of mammalian cells (i.e., the equivalent of about 20,000 inserts of 150 kb). A second, and perhaps more difficult problem, is the absence of a cloning vector that can take advantage of the T4 packaging capability to clone and amplify large DNA fragments. Indeed, since very little is known about the T4 sequences that are needed to initiate T4 packaging, it may be difficult, to construct such a vector.

Burke et al., Science 236, 806-812 (1987), were able to clone large segments of mammalian DNA as minichromosomes in yeast. The DNA to be inserted is cloned into a vector containing a yeast replicating element, a yeast partitioning element or centromere, and yeast telomeres and the resulting chimeric DNA is introduced into yeast by direct DNA transformation. Minichromosomes with inserts of DNA larger than 100 kb were identified. There are two problems with this system. First, yeast clones with inserts of DNA are generated very inefficiently; in the experiments described, about 300 clones were produced per microgram of insert DNA. Second, once clones with inserts of DNA are available, it is difficult to probe and recover the insert DNA. In transformed yeast cells, minichromosomes represent less than 1:1000 of the total DNA of the cell and consequently can only be detected by DNA hybridization techniques. Moreover, within each transformed cell the inserts cannot be amplified, and they can be recovered only, in part, by plasmid rescue into bacteria.

Another system of interest is described by Gaitanaies et al., Gene 46, 1-11 (1986). A lambda vector is described into which DNA can be inserted and which can then be injected into cells. The injected DNA is maintained in cells either integrated into the hosts' chromosome at one copy per cell or extrachromosomally in multiple copies per cell. While this vector can accommodate less than 30 kb of DNA, the amount of DNA in the integrated prophage can be increased significantly by homologous recombination with a second infecting lambda-chimera whose insert overlaps that in the integrated prophage. In this way the segment of contiguous DNA in any prophage can be increased to more than 100 kb and subsequently amplified by inducing the extrachromosomal state of the vector. However, to accomplish this, one needs to have cloned at least several contiguous and overlapping smaller segments of DNA and carry out an arduous process of stringing them together by recombination. Moreover, it will be difficult to recover the large DNA insert once it is constructed, since it will be too big to be packaged into a lambda virus-particle. Direct isolation of the DNA in its extrachromosomal state could also be difficult because of its large size.

### SUMMARY OF THE INVENTION

This invention relates to
1) a vector for cloning DNA fragments comprising the following sequence: bacteriophage P1 loxP site-ampicillin resistance gene-pBR322 ori-bacteriophage P1-pac-bacteriophage P1 loxP site-polylinker cloning site-kanamycin resistance gene-bacteriophage P1 plasmid replicon wherein said sequence is circularized such that the bacteriophage P1 plasmid replicon is attached to the first bacteriophage P1 loxP site so that the sequence reads in a counterclockwise direction.
2) A preferred embodiment of this vector has all the elements recited above as well as a P1 lytic replicon under the control of a lacZ promoter inserted between the polylinker cloning site and the kanamycin resistance gene.
3) A method of cloning DNA fragments as large as 95 kb comprising:
   (a) inserting an exogenous DNA fragment into the polylinker cloning site of the vector of 1) above
   (b) contacting the product of step (a) with bacteriophage P1 pac-cleavage proficient extract and head-tail proficient extract;
   (c) infecting a Cre⁺ gram-negative bacterial strain with the product of step (b); and
   (d) recovering the cloned vector DNA.
4) A method of cloning and controlling amplification of DNA fragments as large as 95 kb comprising (a) inserting exogenous DNA into the polylinker cloning site of the vector containing the P1 lytic replicon under control of a lacZ promotor as defined in 2) above, (b) contacting the product of step (a) with bacteriophage P1-pac-cleavage proficient extract and head-tail proficient extract, (c) infecting Cre⁺, gram-negative bacteria having a lacIq repressor with the product of step (b); (d) derepressing the repressor by adding IPTG to the product of step (c) and (e) recovering the cloned and amplified DNA.
5) A process for in vitro P1 bacteriophage packing of the DNA of the vector as defined in 1) or 2) above having an exogenous DNA fragment inserted therein comprising contacting the DNA of the fragment-containing vector with bacteriophage P1 pac-cleavage proficient extract and head-tail proficient extract wherein said fragment has a size equal to or less than 95 kb. In a preferred embodiment said bacteriphage P1 pac-cleavage proficient and head-tail proficient extracts are prepared from the lysogens NS2962 and NS2961. The packaged DNA can be subsequently used to infect a variety of gram-negative bacteria such as-E. coli.

Cre⁺ gram-negative bacterial strains designated BSS91 and NS2974 wore specifically engineered to be infected and transformed by the vectors of this invention. BS591 does not have a lacIq repressor gene. NS2974, on the other hand, does have a lacIq repressor gene and, thus, once transformed can repress the lacZ promoter until IPTG is added.

### BRIEF DESCRIPTION OF THE DRAWINGS

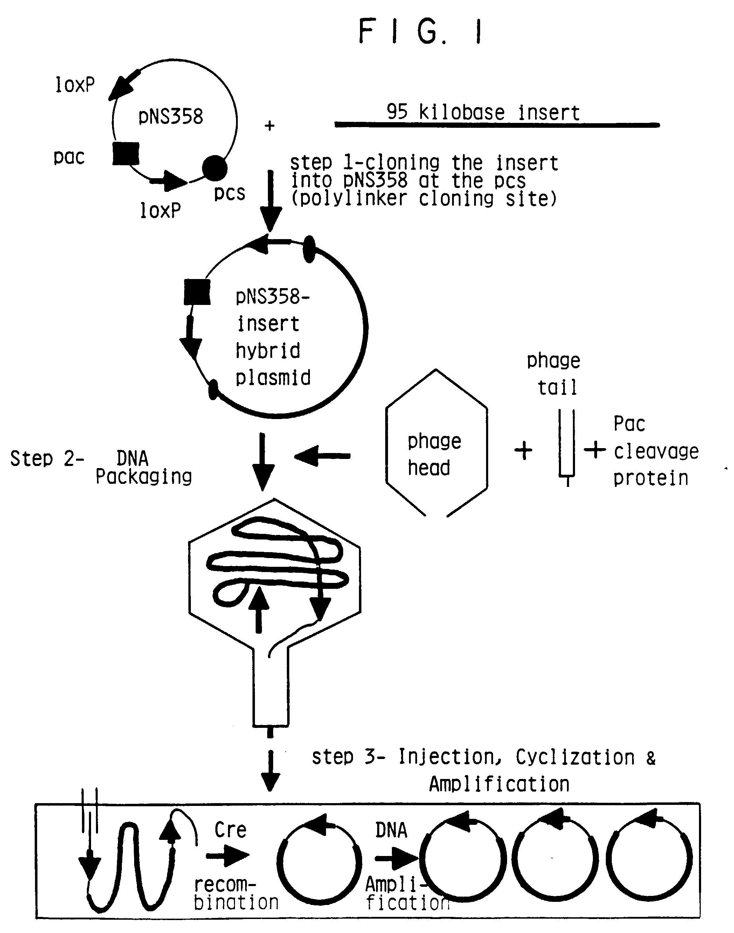

Figure 1 illustrates generally the P1 bacteriophage cloning and amplification system.

Figure 2 maps the P1 genes and elements pertinent to the invention.

Figure 3 illustrates the construction of the vectors of the instant invention.

Figure 4A illustrates that the 600 bp delta-3 pac fragment was labeled at both ends with gamma³²P-dATP.

Figure 4B illustrates the pac-cleavage activity of the pcp⁺ packaging extracts.

Figure 5A illustrates the dissociation of the DNA of pNS358 when acted on by Cre recombinase.

Figure 5B illustrates that vector DNA is acted on by Cre in transformed BS591 bacteria.

Figure 6A shows the structure of pNS364.

Figure 6B illustrates amplification of plasmid pNS364 containing kan-R gene, a P1 plasmid replicon, and a P1 lytic replicon.

Figures 7A-7C and 8A-8B illustrate recovery and characterization of the packaged pNS358 DNA and the E. coli inserts it contained.

### STATEMENT OF DEPOSIT

The following bacteria and plasmids relating to the invention have been deposited in the American Type Culture Collection, 12301 Parklawn Drive, Rockville, Maryland 20852-1776 under the Budapest Treaty.
pNS20 was designated ATCC Accession No. 67666.
pNS42 was designated ATCC Accession No. 67667.
BS591 was designated ATCC Accession No. 53760.
NS2961 was designated ATCC Accession No. 67665.
NS2962 was designated ATCC Accession No. 67664.
NS2974 was designated ATCC Accession No. 53759.

### DETAILED DESCRIPTION OF THE INVENTION

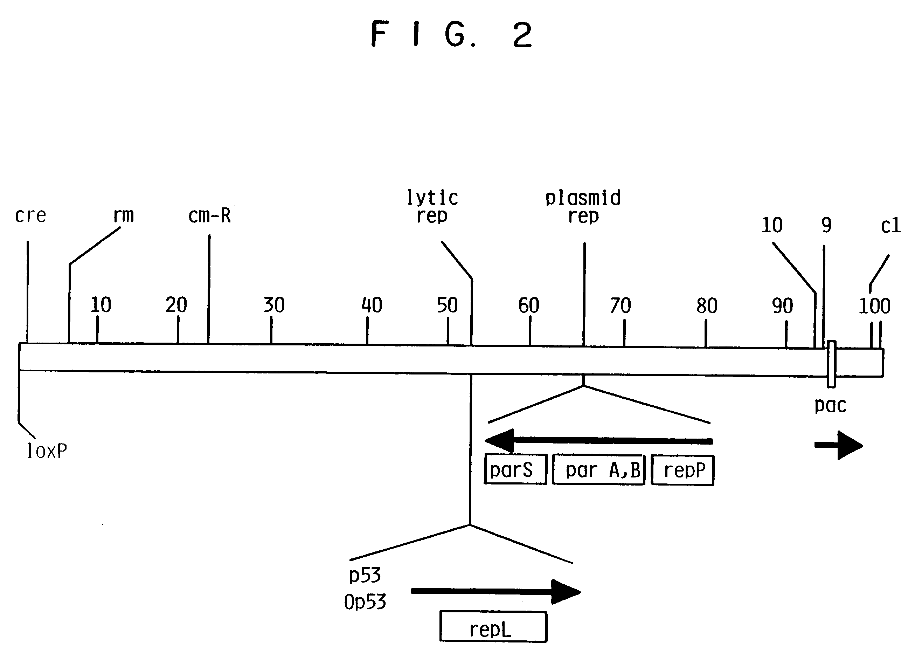

The present invention describes a method which uses an in vitro packaging system of bacteriophage P1 and a novel cloning vector to introduce and control amplification of exogenous DNA inserts as big as 95 kb. Many of the elements that have been used in the construction of this cloning system have been recently reviewed in detail (Yarmolinsky & Sternberg, The Bacteriophage, Bacteriophage P1, Chapter 9, 1988, Plenum Publishing Corporation, 233 Spring St., New York, NY). A map of P1 genes and the loxP-Cre recombination system, the P1 plasmid replicon, the P1 lytic replicon, and the pac site is shown in Figure 2.

The cloning system described here permits the isolation of DNA fragments that are at least twice the size of those that can be obtained by lambda cosmid cloning. This increased size has the following utility:
(1) Genes in the 45-95 kb size range can now be directly cloned and genes in the 25-45 kb size range can be cloned more easily.
(2) Chromosomal "walking" and "jumping" techniques can be speeded up by a factor of at least two and should be more accurate because of the reduced number of contiguous segments that need to be linked together.
(3) As P1 phage appears to be able to inject their DNA into an extensive variety of gram negative bacteria (Table 1), the in vitro P1 packaging system will be useful as a means for the delivery of DNA efficiently to bacteria which otherwise do not take up DNA from solution well.

### The loxP-Cre recombination system

This is a site-specific recombination system that consists of a 34-bp site or DNA sequence (loxP) at which recombination occurs very efficiently and a protein enzyme (Cre) that contacts the site and promotes the recombination. Abremski et al., Cell 32, 1301-1311 (1983), showed that recombination between loxP sites on supercoiled, nicked circle, or linear DNA occurs in the presence of Cre. In the P1 life cycle, Cre can promote recombination between loxP sites that are 100 kb apart. The cre gene has been cloned into a lambda vector and expresses Cre when present as a lambda-Pl:cre prophage in the E. coli chromosome. Sternberg et al., J. Mol. Biol. 187, 197-212, 1986, describe the pHR103 mutant containing the functional cre gene from which the prophage was constructed.

### The P1 plasmid replicon

The P1 plasmid replicon and partition region is responsible for maintaining the P1 prophage as a unit copy extrachromosomal element in a population of lysogenic cells. It contains a replication gene (repP) that encodes a protein which acts at an origin sequence in the DNA to initiate a single round of replication per cell division cycle. Also present in the replicon are two genes, parA and parB, that act at a site parS to faithfully partition the products of replication to daughter cells at cell division.

### The P1 lytic replicon

The P1 vegetative or lytic replicon can replicate DNA to a high copy number within 30 minutes after prophage derepression. This replicon consists of a transcription promoter P53 and a downstream repL gene whose product acts at an origin sequence to promote replication. The replicon is negative regulated by the phage c1 repressor that binds to P53 and prevents transcription of the repL gene.

### Processive headful packaging, pac, and pac recognition proteins (PRP).

The DNA substrate used in the packaging reaction during the viral life cycle is a concatamer consisting of individual units of the P1 chromosome arranged in a head-to-tail manner. Packaging is a four step process: (1) In the first step a unique site, pac, is recognized and cleaved by the PRP protein(s) ; (2) DNA on one side of the cleavage is packaged into an empty phage head until the head has been completely filled; (3) A second cleavage event is then triggered (the "headful" cut) that separates the packaged DNA away from the rest of the concatamer; and (4) Initiation of a second round of DNA packaging from the free end generated by the previous "headful" cut -- hence the term processive headful cutting. Sternberg et al., J. Mol. Biol. 194, 469-479 (1987), have described the cloning of a 161-bp sequence that contains a fully functional pac site. These studies also indicate that pac recognition and cleavage can occur in the absence of phage heads and tails, and is rendered non-functional by mutants in P1 gene 9 (see Figure 2). The ends of the packaged P1 DNA do not contain complementary single-stranded sequences, as do the ends of packaged lambda DNA, and consequently after P1 DNA is injected into a bacterium its cyclization does not occur by strand annealing but rather by recombination between homologous sequences present at the ends of the molecule. Because of this circumstance, any vector that uses P1 packaging, or for that matter any headful packaging mechanism, must devise a means of cyclizing the linear packaged DNA by recombination. In this invention, cyclizing was accomplished (see below) by incorporating the P1 loxP sites into the vector and using the Cre recombinase to cyclize the DNA after injection into Cre⁺, gram-negative bacterial strains.

P1 produces two head sizes, a big head that can accommodate 105-110 kb of DNA, and a small head that can accommodate no more than 40 kb of DNA. Normally the ratio of big to small heads in a P1 wild-type infection is 10:1, however, in the cm-2 mutant of P1 used to prepare the packaging lysates for the invention described herein, the ratio of head sizes is 1:1. To ensure packaging of DNA exclusively into the big phage heads, the DNA must be bigger than that which can be accommodated by the small heads.

### P1 host range

Bacteriophage P1 can adsorb to and inject its DNA into a variety of gram-negative bacteria that otherwise are unable to take up DNA efficiently. A tabulation of bacterial strains to which P1 virions can adsorb and inject DNA is shown in Table 1.

**Table 1**

| Host Range of P1 | | |
|---|---|---|
| Bacterium | P1 DNA Injection | P1 Phage Production |
| Escherichia coli K12,C,B | + | + |
| Shigella dysenteriae | + | + |
| Shigella flexneri | + | |
| Salmonella typhimurium | (-)+ | + |
| Salmonella typhi & abony | + | |
| Klebsiella aerogenes | + | + |
| Klebsiella pneumoniae | + | + |
| Citrobacter freundii | + | + |
| Serratia marcescens | + | (-)+ |
| Enterobacter aerogenes | + | + |
| Enterobacter liquefaciens & cloacae | + | + |
| Erwinia carotovora | + | + |
| Erwinia amylovora | + | + |
| Yersinia pestis & pseudotuberculosis | + | + |
| Pseudomonas putida | - | |
| Pseudomonas aeruginosa | + | (-)+ |
| Pseudomonas amyloderamosa | | (-)++ |
| Flavobacterium sp. M64 | + | - |
| Argobacterium tumefaciens | + | - |
| Acetobacter suboxydans | - | |
| Alcaligenes faecalis | + | - |
| Myxococcus xanthus | + | - |

Also tabulated is the ability of P1 to produce bacteriophages in these strains. With the ability to package DNA into P1 phage in vitro, it should be possible to clone DNA from any of these bacteria directly into a packaging vector, package that DNA into P1 virions, and then inject it back into those bacteria that are proficient for P1 injection. This all can be done without replicating the insert DNA in E. coli which would cause it to lose the methylation pattern that protects it from restriction enzymes when it enters its host of origin.

### Bacterial Strains and Media

E.coli strains DH5 delta-lacU169, W3350, NS439, N99, JM101, JM109, 594 and YMC, or derivatives thereof, may serve as hosts for plasmids and phage P1. DH5 delta-lacU169 was obtained from Dr. Michael Berman, Litton Bionetics, and was a derivative of DH5, a variant of DH1 which was described by Hanahan, J. Mol. Biol. 166, 557-580 (1983). BS591 was DH5 delta-lacU169 (henceforth called DH5) with a lambda-imm434-Pl prophage that was constructed from a pRH103 mutant containing the Cre gene. The pRH103 mutant was described by Sternberg et al., in J. Mol. Biol. 187, 197-212 (1986). BS591 does not contain a lacIq repressor gene. JM101 and JM109 were obtained from New England Biolabs, Beverly, MA 01915, and were described by Yanish-Perron et al., in Gene 33, 102-119 (1985). NS2974 was JM109 and has a lambda-imm434-P1 prophage containing a functional Cre gene and a lacIq repressor. Strains 594 and W3350 were described by Campbell et al., Carnegie Institute of Wash. Yearbook 57, 386 (1987); YMC was described by Dennert et al., J. Mol. Biol. 33, 322-329 (1968), N99 was described by Shimada et al., J. Mol. Biol. 93, 483-503 (1972). NS439 was strain YMC with a trpE 5947 mutation. NS3067 was NS439 and has a lambda-imm434-P1 prophage with a functional cre gene. Media (i.e., L broth and L-amp agar) for bacterial growth were described in Miller, Experiments in Molecular Genetics (Cold Spring Harbor Laboratory, Cold Spring Harbor, NY, 1972).

### Phage Methods

The terms bacteriophage and phage are used interchangeably. P1 phage (P1 bacteriophage, P1) manipulations, including the preparation of phage lysates, making phage genetic crosses, doing phage complementation tests, and bringing about phage lysogenization were described by Sternberg et al., in J. Mol. Biol. 187, 197-212 (1986) and by Sternberg et al., in J. Mol. Biol. 194, 453-468 (1987).

### DNA Preparation and Manipulation

Plasmid DNA smaller than 20 kb was prepared from E. coli according to (1) the rapid method (hereafter referred to as rapid plasmid preparation) described by Holmes & Quigley in Anal. Biochem. 114, 143-197 (1981) or (2) the cesium chloride density gradient method as described by Godson and Vapnek, BMA 299, 516-522 (1973). Plasmid DNA bigger than 20 kb was prepared by the method of Birnboim et al., Nucl. Acids Res. 7, 1513-1523 (1979). Cellular DNA was isolated from E. coli as described by Sternberg et al., J. Mol. Biol. 194, 469-479 (1987). To maintain the size of the DNA above 200 kb, care was taken to avoid shear forces during this isolation procedure. Pulse field agarose gel electrophoresis was carried out as described by Carle et al., Science 232, 65-68 (1986). Restriction enzyme digestion of DNA and DNA ligation with T4 DNA ligase should be done as specified by the vendor, which was in our case, New England Biolabs. All other methods of manipulating DNA were described by Maniatis et al., Molecular Cloning: A Laboratory Manual (Cold Spring Harbor Laboratory, Box 100, Cold Spring Harbor, NY 1982).

### Southern Analysis

DNA was transferred to nitrocellulose membranes from agarose gels and probed with specific labeled DNA fragments or plasmids according to the method of Southern, J. Mol. Biol. 98, 503-507 (1975), henceforth referred to as "Southern analysis". DNA was sequenced using the dideoxy nucleotide procedure of Sanger et al., Proc. Natl. Acad. Sci., USA, 74, 5463-5467 (1977).

### Restriction Analysis

Five hundred nanograms of DNA was digested with 1-2 units of restriction enzyme for 2 hours at 37°C in 20 microliters of a buffer specified by New England Biolabs (Beverly, MA 01915), from whom the enzyme was purchased. The reaction was stopped by adding 3 microliters of a solution of 0.25% bromophenol blue, 0.25% xylene cyanol, and 40% (w/v) sucrose, followed by heating the reaction to 70°C for 5 minutes. The samples were loaded into the slots of a 1% agarose gel (15x15 cm) that was submerged in a Tris-Borate-EDTA buffer (0.089 M Tris-Borate, 0.089 boric acid, 0.002 M EDTA). Electrophoresis was carried out at 20 milliamps (30 volts) until the xylene cyanol band migrated two-thirds of the way into the gel, usually about 12 hours at room temperature. The gel was then stained in a solution 0.5 micrograms/ml of ethidium bromide for 30 minutes, and a picture of the gel was taken using a transmitted UV light source (320 nm) and polaroid type 57 film.

### Treatment of DNA with alkaline phosphatase (AP)

After DNA was digested by restriction enzymes as described in the previous section, it was heated to 70°C for 5 minutes. The reaction was cooled to 37°C, brought to 100 microliters with a 10 mM Tris-HCl pH 8.0 solution, and then incubated with 0.01 units of calf intestine alkaline phosphatase for 1 hour at 37°C. The reaction was extracted 4 times with an equal volume of phenol and once with an equal volume of chloroform-isoamyl alcohol (24:1) and the DNA then precipitated in 1 M LiCl₂ with 2 volumes of ethanol at -20°C for 3 hours.

### Ligation and insertion of fragments into vectors

Ligation reactions were carried out in a volume of 20 microliters containing 6 mM Tris-HCl pH 7.5, 5 mM MgCl₂, 5 mM dithiothreitol, 1 mM ATP, 100 micrograms/ml bovine serum albumin. For reactions involving the insertion of fragments into plasmids that were to be recovered by transformation into competent bacteria, 100 ng of digested vector DNA and 100-400 ng of insert fragment were used. For reactions involving plasmid vectors that were to be recovered by in vitro phage packaging, 500 ng of vector DNA and 1-5 micrograms of insert fragment were used. 400 units of T4 ligase were added for each reaction and the reactions were incubated for 20 hours at 16°C. The reactions were stopped by heating to 70°C for 10 minutes.

### Selection of transformants

E. coli strains were transformed according to (1) a method of Mandel and Higa, J. Mol. Biol. 53, 159-162 (1970) or (2) a method of Hanahan, J. Mol. Biol. 166, 557-580 (1983) when high efficiency was required. Transformed cells in a volume of 0.1 ml were diluted to 1 ml with L-broth and incubated for 1 hour at 37°C. 100-200 microliters of the culture was spread onto L agar plates containing either kanamycin (25 microliters/ml) or ampicillin (150 micrograms/ml) and the plates incubated for 16 hours at 37°C. Colonies were then scored. Amp-R transformants were tested for growth on L-agar plates with kanamycin and vice versa.

### Construction of the plasmid vector. pNS358-lyt

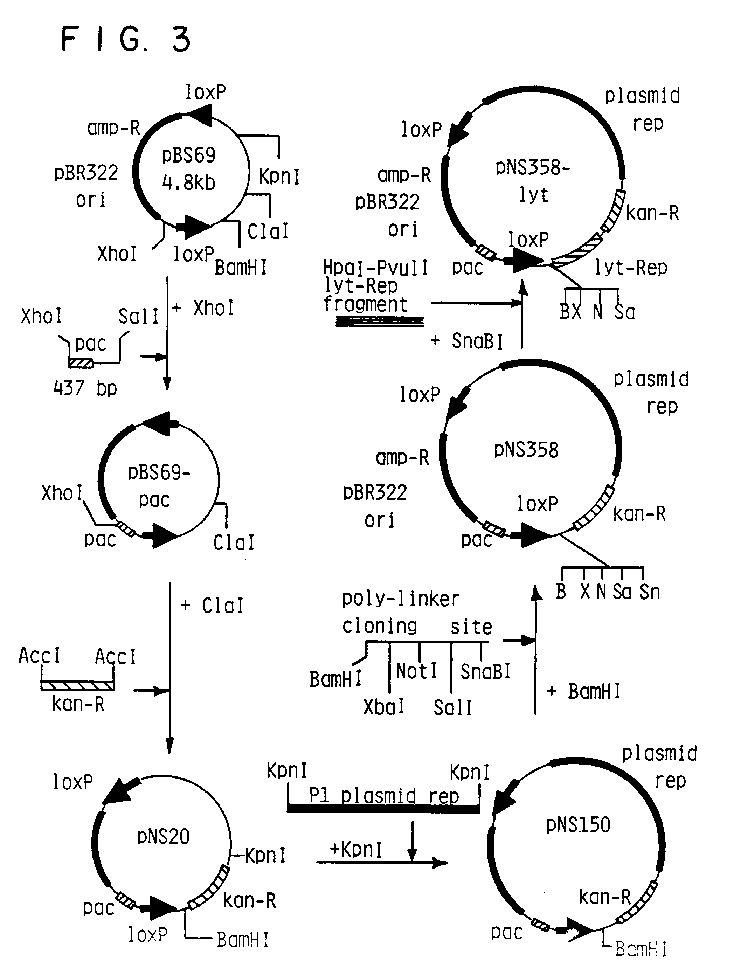

A flow sheet describing the various steps in the construction of the vector is shown in Figure 3. The starting plasmid was pBS69. It was a 4.9 kb plasmid containing two loxP recombination sites orientated in the same direction. In the presence of Cre protein, recombination occurs between these sites to generate two circles of DNA, each containing one loxP site and one of the two regions of DNA between the loxP sites in pBS69. One of these circularized regions, henceforth called the amp-R domain, contained the gene that codes for resistance to the drug ampicillin and the DNA replicating system (replicon or origin of replication [ori]) of the plasmid pBR322 (hereinafter pBR322 ori). The other circularized region between the loxP sites of pBS69 contained the yeast leu2 gene.

### (a) Insertion of the P1 packaging site. pac. into pBS69

The P1 sequences necessary for the intiation of DNA packaging were localized to a 161 bp segment of DNA (pac site). This segment of DNA represented the P1 sequences remaining in the double-deletion mutant (delta-3 - delta-20) described by Sternberg et al., J. Mol. Biol. 194, 469-479 (1981). Delta-3 - delta-20 DNA was digested with restriction enzymes XhoI and SalI and a 437 bp fragment containing the 161 bp pac site was isolated and ligated to the unique XhoI site in the amp-R domain of pBS69 DNA. The resulting pBS69-pac plasmid contained pac oriented such that packaging would occur countercloskwise as the plasmid is drawn in Figure 3. In addition to pac, the 437 bp insert contained the tetracycline gene sequences located between pBR322 map coordinates 375 and 651. Also, the BamHI site, present in the 437 bp DNA fragment and located between pac and the pBR322 sequences in the plasmid DNA, was deleted. Removal of the BamHI site ensured that the pBS69-pac plasmid would have only the single pBS69 BamHI site.

### (b) Insertion of the Tn903 Kanamycin-resistance gene into pBS69-pac to generate pNS20

The Tn903 kanamycin resistance (kan-R) gene was isolated as a 1.3 kb AccI fragment from plasmid puc4K, which was purchased from PL-Biochemicals (Milwaukee, WI). pBS69-pac DNA was digested with the restriction enzyme ClaI, which cleaved that DNA once within the yeast leu2 gene. Equal amounts (100 ng) of that digested plasmid DNA and the kan-R gene fragment were mixed in 20 microliters and ligated overnight at 16°C. The ligation mix was used to transform strain DH5 bacteria and kan-R, amp-R transformants selected. Analyses of rapid plasmid preparations made from these transformants indicated that they all contained the kan-R gene at the ClaI site of pBS69-pac. The plasmid chosen for all subsequent manipulations, henceforth designated pNS20, contained the kan-R fragment oriented such that the direction of kan transcription was counterclockwise (Figure 3). The region flanked by loxP sites containing this kan-R gene will henceforth be designated the kan-R domain.

### (c) Insertion of the P1 plasmid replicon into pNS20 to generate pNS150

A 7 kb KpnI fragment of DNA, located between P1 map coordinates 59 and 66 (Figure 2), was isolated after digesting the DNA from P1 phage with KpnI. It was ligated into the unique KpnI site of pNS20, and its presence in the plasmid was confirmed by analyzing restriction enzyme digests of rapid plasmid preparations. The particular construct chosen for further manipulation was designated pNS150 and contained the P1 DNA from map coordinate 59 to 66 oriented counterclockwise in the plasmid. The 7 kb P1 insert contained not only the plasmid replication system, but also its partition system (Yarmolinsky and Sternberg, in "The Bacteriophages", Chapter 9, 1988).

### (d) Insertion of a polylinker cloning site into pNS150 DNA to generate pNS358

Two 31 base oligodeoxynucleotides with complementary sequences were synthesized by the phosphoramidite method. 10 micrograms of each oligonucleotide in a volume of 100 microliters (10 mM Tris-HCl buffer at pH 8.0, 1 mM EDTA) were annealed to each other at 70°C for 10 minutes.
The resulting double stranded fragment is shown below:

GATCCTCTAGAGCGGCCGCGTCGACTACGTA
GAGATCTCGCCGGCGCAGCTGATGCATCTAG
------________------______
XbaI NotI SalI SnaBI

This fragment of DNA contained, from left to right, the following restriction endonuclease sites: XbaI (X) - AGATCT; NotI (N) - CGCCGGCG; SalI (Sa) - CAGCTG; SnaBI (Sn) - ATGCAT. None of these sites were present in the DNA of pNS150. The fragment also contained single-stranded GATC ends that were complementary to the ends generated by BamHI digestion. Because the nucleotides 3' to the GATC ends of the fragment were different, ligation of this fragment to DNA with BamHI ends regenerated a BamHI site on one side of the insert but not on the other. The fragment was ligated to BamHI-cleaved pNS150 DNA and the presence of the insert was confirmed by the ability of the plasmid to be cleaved only once by each of five restriction enzymes BamHI, XbaI, NotI, SalI, and SnaB1. The particular construct chosen for further manipulation was designated pNS358 and had the polylinker from BamHI to SnaBI oriented counterclockwise on the plasmid (Figure 3).

### (e) Insertion of the P1 lytic replicon into pNS358 to generate pNS358-lyt

The P1 lytic replicon was cloned as a 1.9 kb AsuII fragment (P1 map coordinates 53-55) in which the normal P53 promoter was replaced by the lacZ promoter. Thus, the P1 lytic replicon was placed under the control of the lacZ promoter. The lacZ promoter is in turn controlled by the lacIq repressor. In the presence of IPTG (isopropyl-beta-D-galactoside) the lacIq repressor is derepressed and the replicon becomes functional. The lacZ promoter-regulated P1 lytic replicon was isolated as a PvuII-HpaI fragment from plasmid pNS42 and ligated to SnaBI-digested pNS358 DNA, to generate the pNS358-lyt plasmid. The replicon (P1 map coordinates 53-55) was oriented counterclockwise in the plasmid.

### Construction and properties of phage and lysogens used in generating P1 packaging extracts

The phage used to construct lysogenic bacteria (lysogens) for the preparation of P1 packaging extracts were the quadruple mutants P1rm⁻cm-2c1.100 9.16 or P1rm-cm-2c1.100 10.1. The properties of the individual mutations, and the reason for their incorporation in the final phage, are discussed below.
(a) c1.100. This mutation, first described by Rosner, Virology, 48, 679-689 (1972), renders the phage c1 repressor temperature sensitive. Thus lysogens containing this P1 prophage grow normally at temperatures below 33°C but are induced to enter the lytic cycle and produce phage at higher temperatures. For example, if the temperature of the culture is raised from 33°C to 40°C, and the culture is held at that temperature, cell lysis starts to occur within 50 minutes of the shift and about 100 phage/cell are released.
(b) rm⁻. This mutation, first described by Glover et al., Genet. Res., 4, 480-482 (1963) renders the restriction and modification system of the virus inactive. It was incorporated into the phage used here so that extracts made from induced P1 lysogens would not contain a restriction endonuclease activity that could destroy added DNA before it might be packaged.
(c) cm-2. This mutation is described in Iida and Arber, Mol. Gen. Genet., 153, 259-269 (1977) and is a gross chromosomal rearrangement in which a Tn9 transposon, with its chloramphenicol resistance (cm-R) gene, was inserted at P1 map coordinate 24 and a 10 kb portion of P1 DNA between map coordinates 24 and 33 was deleted. The mutation renders the phage partially lysis defective so that following induction of a lysogen, bacteria can be harvested at later times than is otherwise possible. Another property of this mutant is that it produces more small-headed phage variants than does wild-type P1.
(d) am9.16 and am10.1. P1 amber (am) mutant 10.1 contains a nonsense mutation in P1 gene 10 (see Figure 2) and is defective for all "late" phage protein synthesis. It produces a normal amount of pac-cleavage activity. P1 amber mutant 9.16 contains a nonsense mutation in P1 gene 9 and is defective for the production of pac-cleavage activity. It produces phage morphogenesis proteins normally, including phage heads and tails. While extracts prepared from either one of these mutants are not expected to be able to package DNA in vitro, both extracts together should have all the needed components for packaging.

The quadruple phage mutants were constructed in a two-step process. First, phages P1 cm-2 and either P1 c1.100 9.16 or P1 c1.100 10.1 were crossed and recombinant phage containing the three mutations, P1 cm-2 c1.100 9.16 or P1 cm-2 c1.100 10.1, were selected. The phage produced by this cross were used to generate plaques on a bacterial lawn of strain YMC and individual plaques screened for the following three properties: (1) the ability to produce lysogens that were cm-R; (2) temperature sensitivity; and (3) the ability to make plaques on bacterial strain YMC but not on strain N99. The former strain contained an amber suppressor while the latter did not. To confirm that amber mutant recombinants contained the correct amber mutation, complementation experiments with control 9.16 and 10.1 phages were carried out. Amber mutants in the same gene will not complement each other for phage production but those in different genes, like genes 9 and 10, will. The complementation tests confirmed that the amber mutations in the triple mutants were those expected based on the phage used to generate those mutants. The triple mutants were next crossed with P1rm⁻ and the quadruple mutants were identified as having all of the properties of the triple mutants plus the inability to restrict the growth of phage lambda when present as a prophage in any E. coli strain.

The quadruple P1 mutants were used to lysogenize strain N99 and those lysogens, designated NS2961 and NS2962, respectively, for the 9.16 and 10.1 mutants, were used to prepare P1 extracts for the in vitro packaging reaction. Furthermore, these P1 mutants can also be used to lysogenize any bacterial strain proficient for their uptake.

### Preparation of P1 packaging extracts

### (a) Preparation of the 10.1 or pac-cleavage proficient (pcp⁺) extract

One liter of L broth was inoculated with a colony of strain NS2962 bacteria and the culture grown at 32°C to an OD650 of 0.8. The temperature of the culture was then rapidly raised to 42°C by swirling it in a 90°C water bath and growth continued with vigorous aeration at 42°C for 15 minutes. The temperature of the culture was then lowered by placing it in a 38°C water bath and vigorous aeration continued for an additional 165 minutes. The bacterial suspension was then rapidly chilled to 4°C in an ice slurry and pelleted by centrifugation at 6000 x g for 10 minutes at 4°C in a Sorvall GSA rotor. The cell pellet was resuspended in 2 ml of a buffer consisting of 20 mM Tris-HCl pH8.0, 1 mM EDTA, 50 mM NaCl, and 1 mM phenyl methyl sulfonyl fluoride (PMSF). The resuspended cells were sonicated on ice with the medium tip of a Branson sonifier at setting 5 for five 40-second intervals. Between each sonification burst the sample was placed on ice for 60 seconds. The sonicated extract was then centrifuged for 30 minutes at 34,000 x g and the supernatant was then divided into 10 microliter aliquots and stored frozen at -80°C. These extracts were usually stable to several rounds of freeze-and-thaw.

### Pac-cleavage activity of the pcp⁺ packaging extract

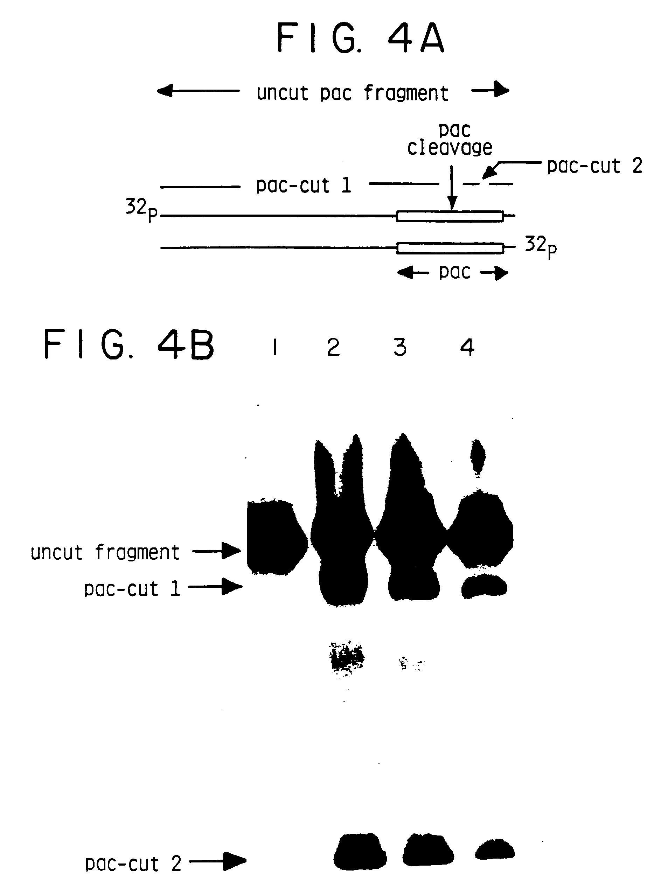

To measure pac cleavage, the 600 bp delta-3 pac fragment was used, which contained the pac site at its right end (Figure 4A; Sternberg and Coulby, J. Mol. Biol. 194, 469-479 (1987)). The fragment was labelled at both ends with gamma³²P-dATP by using polynucleotide kinase and then incubated with various aliquots of the pcp+ extract. The reaction was carried out in 15 microliters of 10 mM Tris-HCl, pH 8.0, 50 mM NaCl, 10 mM MgCl₂, 1 mM DTT, 1 mM ATP, 10 micrograms sonicated calf thymus DNA with 1 microliter of labelled pac fragment (12 micrograms/ml) and either no extract or 0.01 to 1 microliter of the pcp⁺ extract (3.4 milligrams protein/ml). The reaction mixture was incubated for 15 minutes at 30°C and the reaction stopped by adding SDS to a concentration of 0.2% and heating the mixture at 70°C for 5 minutes. The products of the reaction were analyzed by polyacrylamide gel electrophoresis. Samples were loaded into the slots of a vertical 5% gel and electrophoresed for 4 hours at 150 volts. The gel was then dried and exposed to Kodak XRP film overnight. The results, illustrated in Figure 4B, show that 1 microliter of the extract (lane 2) cleaved about 20% of the fragment, generating two new fragments not seen in the lane without added extract (lane 1). A ten-fold dilution of the extract (lane 3) reduced the amount of cleaved fragment only marginally, but an additional ten-fold dilution of extract (lane 4) reduced the efficiency of cutting to about 5%.

### (b) Preparation of the 9.16 or head-tail proficient (htp⁺) extract

One liter of L broth was inoculated with a colony of strain NS2961 bacteria and the culture grown and induced as described above in (a). After shifting the temperature to 42°C and incubating the culture at that temperature for 15 minutes, the temperature was shifted back to 38°C followed by an incubation period of 45 minutes. The culture was divided in half and the cells pelleted as described above in (a). Bacterial cells from one-half of the culture were resuspended in 1 ml of a buffer containing 20 mM Tris-HCl, pH 8.0, 50 mM NaCl, 1 mM EDTA, 5 mM MgCl₂, 1 mM PMSF, and 5 mM beta-mercaptoethanol and sonicated as described above in (a). Aliquots of 100 microliters were directly frozen at -80°C. Cells from the other half of the culture were resuspended in 1 ml of a solution of 50 mM Tris-HCl buffer at pH 7.4, 10% sucrose and were then frozen and thawed twice. Freezing was carried out in liquid nitrogen and the frozen cells were thawed at room temperature. An 8 micromolar solution of egg white lysozyme (10 milligrams/ml) was then added to the thawed cells and after 15 minutes at 4°C, 200 microliters of the buffer used for the sonicated htp⁺ extract was added. After all of the components were gently mixed at 4°C for 5 minutes, 100 microliter aliquots of the extract were frozen at -80°C.

It should be noted that bacterial strain NS2962 was induced for a much longer period of time than was strain NS2961 before the cells were harvested. This was so because the 10.1 amber mutation in the former strain inhibited cell lysis much more dramatically than did the cm-2 mutation which was present in both cell lines. Thus, the induced NS2962 lysogen did not lyse for as long as 4 hours after induction while the induced NS2961 lysogen began to lyse about 80-100 minutes after induction. Indeed, care was taken to rapidly cool the induced NS2961 cells before they were pelleted by centrifugation to avoid lysing the cells during the centrifugation step.

### Demonstration that vector DNA is acted on by Cre in transformed BS591 bacteria

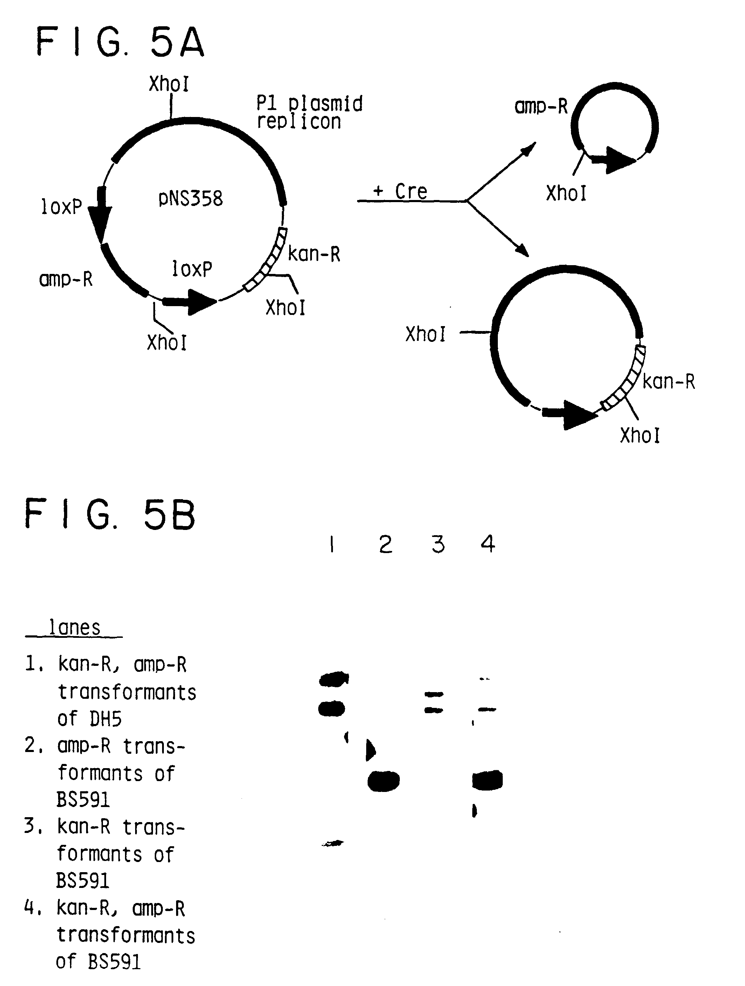

In order to demonstrate that the vectors constructed here would be processed as expected when introduced into cells containing the Cre recombinase, pNS20 and pNS358 were introduced into E. coli strains DH5 and BS591. DH5 did not contain a functional cre gene whereas BS591 did contain a functional cre gene. The results of transformation to amp-R and kan-R are shown in Table 2 and indicate that Cre efficiently dissociated the two domains of both of these plasmids in cells and that each domain must contain a replicon if it was to be recovered. (See Figure 5A.)

**Table 2**

| | | Strain transformed | | | |
|---|---|---|---|---|---|
| Plasmid | Replicon in kan-R domain | BS591 (cre⁺) | | DH5 (cre⁻) | |
| | | amp | kan | amp | kan |
| pNS20 | None | 1.4 | 0.002 | 1.2 | 1.0 |
| pNS358 | P1 plasmid | 0.6 | 0.5 | 0.4 | 0.5 |

When strain BS591 was transformed with pNS20, which carried a replicon only in the amp-R domain, the efficiency of amp-R transformation was about 1000 times higher than was the efficiency of kan-R transformation. The results of transformation of strain DH5 support the conclusion that this was due to the separation of the two domains by Cre-mediated recombination between loxP sites. In this case, the efficiency of amp-R and kan-R transformation with pNS20 was the same. If the kan-R domain carried a replicon, as was the case for plasmid pNS358, then both kan-R and amp-R genes are equally represented among the transformants of strain BS591. Moreover, about 20-30% of the kan-R transformants of BS591 were ampicillin sensitive and an equal number of the amp-R transformants were kanamycin sensitive. As expected, all the transformants of DH5 (Cre⁻) by pNS358 DNA were both amp-R and kan-R. One unit of transformation in these experiments was meant to represent 10⁶ transformants per microgram of DNA used.

Physical evidence for the dissociation of the DNA of pNS358 in BS591 (Figure 5B) came from the restriction analysis of rapid plasmid preparation of DNA derived from the transformants of BS591 and DH5. All DNAs were digested with the restriction enzyme XhoI and the number of fragments resulting were determined by agarose gel electrophoresis and Southern hybridization. Plasmid DNA derived from the amp-R kan-R transformants of DH5 (lane 1) was indistinguishable from the original pNS358 plasmid. They both contained the expected 3 XhoI fragments. In contrast, plasmid DNA from amp-R, kan-S transformants of BS591 contained DNA with only a single XhoI site (lane 2) while plasmid DNA from kan-R, amp-S transformants of BS591 contained two XhoI fragments (lane 3). Plasmid DNA from amp-R kan-R transformants (lane 4) of BS591 contained all three species of DNA, i.e., that like pNS358, amp-R domain plasmid DNA with a single XhoI site, and kan-R domain plasmid DNA with two XhoI sites. As expected, plasmid DNA with just the kan-R domain of pNS358 was recovered in much smaller amounts (i.e., the DNA bands were much lighter) than was either pNS358 DNA or amp-R plasmid DNA. The P1 plasmid replicon in the kan-R domain replicated DNA to a much lower copy number than did the pBR322 replicon in the amp-R domain.

### Demonstration of DNA packaging by pcp⁺ and htp⁺ extracts

### The packaging of ligated vector (pNS358) DNA

Five micrograms of BamHI-digested pNS358 DNA (100 micrograms/ml) was ligated overnight to generate concatamers that ranged in size from 50 kb to 150 kb. Thirty ng of this DNA was incubated with 0.2 microliter of the pcp+ extract as described in the previous section except that the incubation period was for 60 minutes, not 15 minutes. At the end of the incubation period, 2 microliters of a buffer containing 6 mM Tris-HCl, pH 7.4, 15 mM ATP, 16 mM MgCl₂, 60 mM spermidine, 60 mM putrescine and 30 mM beta-mercaptoethanol was added to the reaction along with 8 microliters of the freeze-thawed htp⁺ extract. The resulting mixture was incubated an additional 60 minutes at 30°C. At the end of this period, the reaction was diluted to 150 microliters with TMG (10 mM Tris-HCl, pH 7.5, 10 mM MgSO₄, 0.1% gelatin) and DNaseI was added to a concentration of 10 micrograms/ml to destroy all of the unpackaged DNA. Five microliters of the above packaging reaction was added to 10⁸ bacterial cells of strain BS591 and the resulting mixture was incubated for a period of 10 minutes at 30°C to allow phage adsorption to occur. The resulting infected cells were diluted with L Broth to 2 ml and grown for 1 hour at 37°C, pelleted by centrifugation, and then spread on L-amp agar plates. After overnight incubation, the plates contained 2208 colonies. This corresponded to an efficiency of packaging of 2 x 10⁶ packaged DNA molecules/microgram of added vector DNA. If the bacterial strain used to assay the packaged vector was DH5 (Cre⁻), then the number of amp-R colonies detected decreased about 40-fold, indicating that Cre-mediated recombination between loxP sites was needed to cyclize the injected linear DNA. If either of the two extracts was left out of the packaging reaction, then no amp-R colonies were detected, indicating that both extracts were needed for packaging. If the sonicated htp⁺ extract was used instead of the freeze-thawed htp⁺, the packaging efficiency was unaltered. However, as the sonicated extract proved to be much less stable than the freeze-thawed extract, all subsequent experiments employed the freeze-thawed extract. Finally, analysis of the amp-R colonies indicated that 50% (25/50) were also kan-R, indicating that frequently both antibiotic resistance domains of pNS358 were packaged.

### Infection of bacteria with vectors

One hundred microliters of an overnight culture of bacteria (2-3 x 10⁹ cells/ml) were incubated with an aliquot of a phage lysate or an in vitro packaging reaction at 30°C for 10 minutes. If phage were to be assayed, 3.0 ml of molten L-top agar (0.7% agar) at 55°C was added to the infected cells and the mixture spread on an L-agar plate. After the top agar was allowed to solidify for 5 minutes at room temperature, the plates were incubated for 16 hours at 37°C and plaques were scored. If antibiotic-resistant cells were to be assayed, the infected cells were spread on antibiotic-containing L-agar plates and these plates were incubated for 16 hours at 37°C.

### Recovery of exogenous DNA fragments cloned into pNS358 DNA following in vitro packaging and transformation of Cre⁺ cells

Plasmid with cloned inserts of E. coli DNA was recovered in two ways: (1) Plasmid DNA was isolated from 1 liter of cells grown overnight to 2-3 x 10⁹ cells/ml by the alkali lysis protocol of Birnboim et al., Nucl. Acids Res. 7, 1513-1523 (1979) and by cesium chloride ethidium bromide equilibrium centrifugation, which was described by Maniatis et al., Molecular Cloning: A Laboratory Manual (Cold Spring Harbor Laboratory, Box 100, Cold Spring Harbor, NY, 1982). After the DNA was removed from the gradient, it was extracted four times with cesium chloride-saturated isopropanol, dialyzed against 1 liter of a 10 mM Tris-HCl, pH 8.0, 1 mM EDTA solution for 5 hours at 4°C, extracted twice with phenol, and then precipitated with 2 volumes of ethanol in 1 M LiCl₂. (2) The plasmid with cloned insert DNA was recovered from transformed cells in phage particles by infecting those cells with P1 phage. The infecting P1 DNA recombined with the resident plasmid in the cell by a Cre-mediated recombination process involving loxP sites on both DNAs. This event placed a P1 pac site adjacent to the cloned insert-plasmid and thereby permitted its packaging into phage particles. One hundred microliters of an overnight culture of transformed cells (about 2 x 10⁸ cells) were incubated with 6 x 10⁸ P1 phage for 10 minutes at 30°C and were then diluted to 5 ml with L-Broth. The infected cells were vigorously shaken at 37°C until cell lysis was observed (about 90 minutes). The number of phage containing the cloned fragment along with the kan-R domain of pNS358 was assessed by infecting BS591 with an aliquot of the lysate and then measuring the number of kan-R cells produced. Plaque-forming phage in the lysate were measured as previously described. Usually, the ratio of kan-R phage to plaque forming phage in these lysates was about 0.1-1%.

### Amplification of a plasmid containing a kan-R gene, a P1 plasmid replicon, and a P1 lytic replicon, pNS364

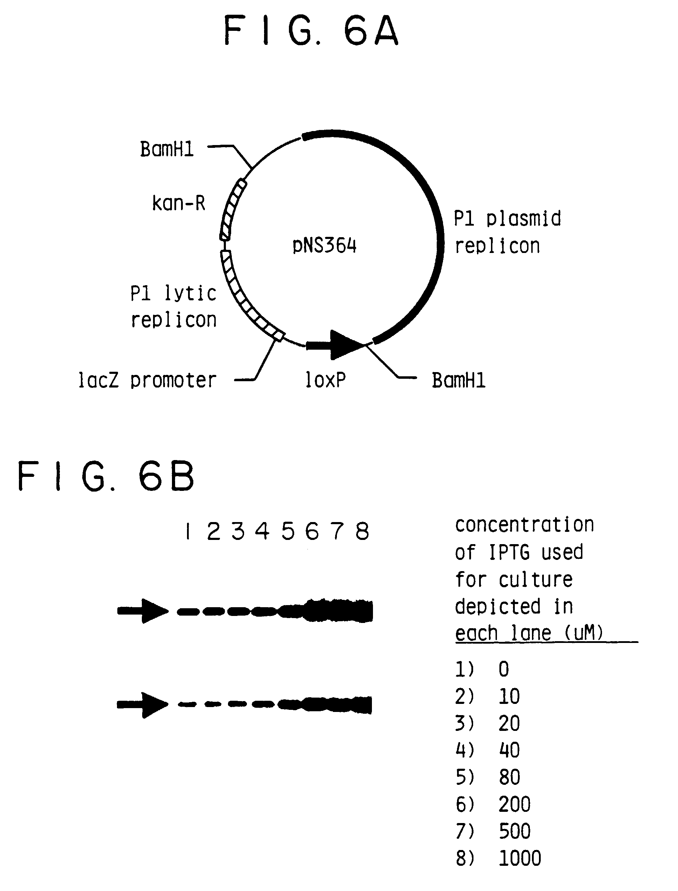

The structure of this plasmid is illustrated in Figure 6A. We used it here to illustrate that a cloned P1 lytic replicon that is regulated by the lacZ gene promoter can be used to easily amplify the number of plasmid copies in the cell. Strain JM109 (lacIq) was transformed with pNS364 DNA and the transformed cells were grown in L-broth. Under these conditions, the plasmid was expected to be maintained by the plasmid replicon at one copy per cell chromosome. When IPTG was added to the media, the lacIq repressor was inactivated, thus, activating the lacZ promoter and the P1 lytic replicon. Sixty ml of a culture of JM109 (pNS364) was grown to a density of 5 x 10⁷ cells/ml in L-broth at 37°C and then divided into 8 aliquots of 5 ml each. One aliquot was grown for an additional 3 hours at 37°C in L-broth, while each of the other seven aliquots was grown in L-broth for 3 hours with IPTG, ranging in concentration from 10 micromolar to 1 mM. Total cellular DNA was isolated from each of the cultures, digested with the restriction enzyme BamHI (which cleaved pNS364 into two fragments), and analyzed by Southern hybridization. The results (Figure 6B) indicated that as the concentration of IPTG in the media increased, so did the plasmid copy number. At 1 mM IPTG, the copy number of the plasmid was 30-40 higher than was the copy number of the plasmid in media lacking IPTG (compare lanes 1 and 8).

### EXAMPLE 1

### Inserting fragments of E. coli DNA into pNS358, packaging the chimeric vector DNA, transforming bacteria with the packaged chimeric vector, selecting transformed bacteria and recovering the inserted DNA fragments

Two micrograms of pNS358 DNA was digested with either BamHI or NotI restriction enzymes. Each of these enzymes cleaved the vector DNA once in the polylinker cloning site. The cleaved DNAs were then treated with alkaline phosphatase to prevent religation of the cleaved ends in the subsequent ligation reaction. The source of insert DNA was E. coli strain W3350 and the isolated DNA was greater than 200 kb in size. Prior to inserting any of the DNA in the vector, it was digested with either Sau3aI to an average size of 20-50 kb or to completion with restriction enzyme NotI. Approximately equal amounts (200 ng) of the vector DNA and E. coli DNAs (BamHI-digested vector with Sau3aI-digested E. coli DNA or NotI-digested vector and NotI-digested E. coli DNA) were mixed in a volume of 20 microliters and ligated overnight. One microliter of this ligation mix was packaged as described in the section entitled "Demonstration of packaging of DNA by packaging extracts pcp⁺ and htp⁺", and used to transform E. coli strain BS591 by infection as described in the section "Infection of bacteria with vectors". The resulting transformed bacteria were detected as kan-R colonies as shown in Table 3.

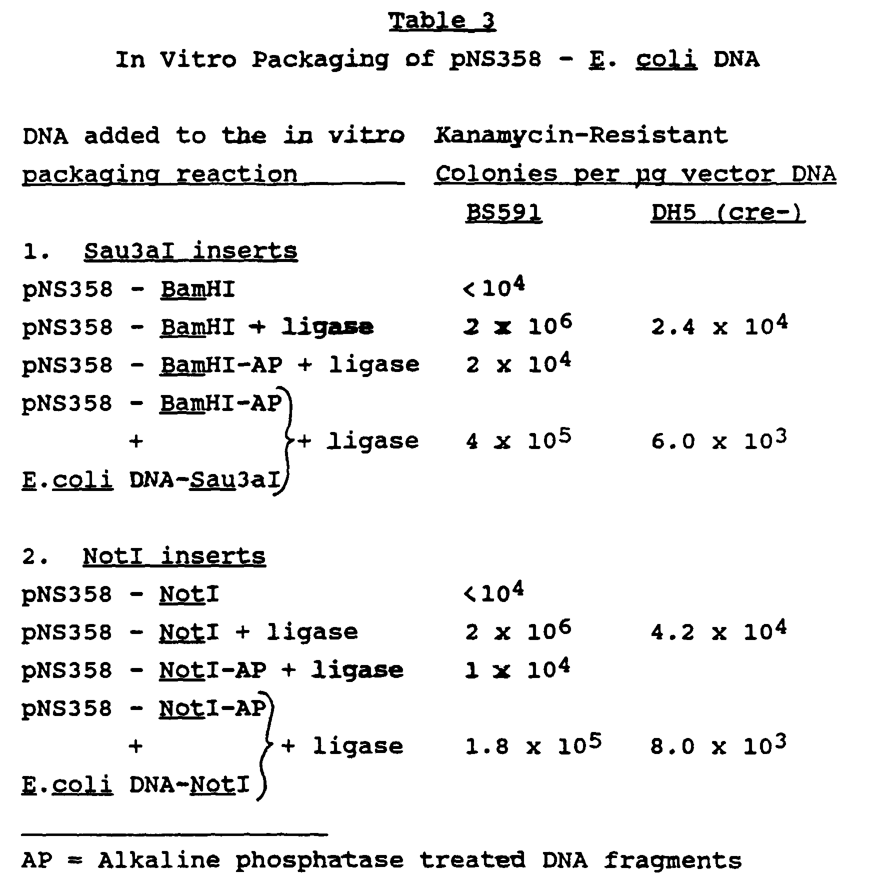

The efficiency of packaging BamHI or NotI-digested vector DNA that had not been treated with alkaline phosphatase was about 2 x 10⁶ packaged DNA molecules/microgram of added vector DNA. Treatment of the vector DNA with alkaline phosphatase reduced the recovery of DNA after packaging about 100 to 200-fold, but Sau3aI- or Noti-digested E. coli DNA may be added to the ligation reaction to partially restore that loss. The interpretation of these results was that the phosphatase-treated vector DNA cannot be ligated to large concatamers and consequently cannot be packaged efficiently. Adding E. coli DNA inserts to the vector DNA permitted it to reach a size that can be packaged. Depending on the particular packaging reaction, 50-90% of kan-R colonies were amp-S, indicating that they lacked the amp-R domain of pNS358.

### Recovery and characterization of the packaged pNS358 DNA and the E. coli inserts it contained

### (a) The Sau3aI clones

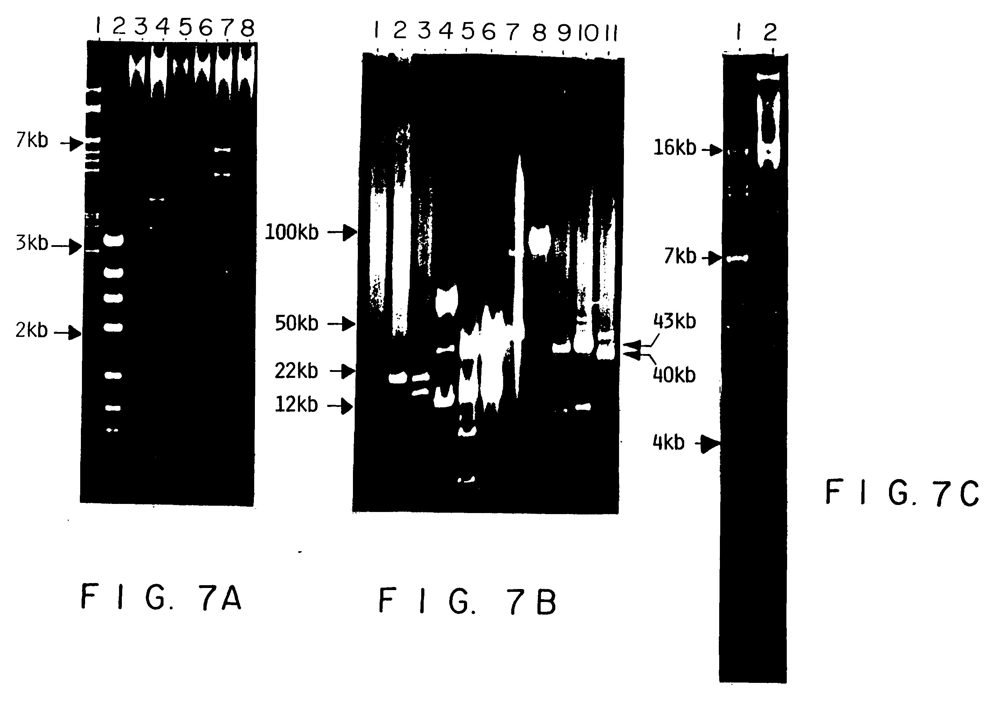

The plasmid DNA in each of the cultures grown from the 10 separate colonies generated by packaging the Sau3aI-digested E. coli DNA inserted in pNS358 was isolated by the alkali procedure of Birnboim et al., Nucl. Acids Res. 7, 1513-1523 (1979), and analyzed by restriction enzyme digestion. The DNA of two of the plasmids was identical to the kan-R domain of pNS358 DNA, but the rest contained substantially more DNA than was present in the vector, including many new restriction fragments. Digests of the vector (lane 3) and of five of the vector-Sau3aI chimeras (lanes 4-8) with the restriction enzymes BglII, XhoI, PvuII and EcoV are shown in Figure 7A. It appeared that new DNA fragments seen in lanes 4-8 come from the insertion of E. coli Sau3aI fragments. Lanes 1 and 2 of Figure 7A contained DNA size markers. The biggest plasmid isolated was 38-40 kb. Pulse field gel analysis indicated that it contained two NotI fragments of 22 kb and 17 kb (Figure 7B, lane 3). Lane 1 contained packaged vector DNA and lane 2 contained a second Sau3aI-chimera digested with NotI. These results suggest that most, if not all of the Sau3aI inserts were packaged in small-headed (P1S) particles, whose capacity is no greater than 40 kb.

### (b) The NotI clones

The recent NotI restriction map of E. coli DNA described by Smith et al., Science 236, 1448-1453 (1987), shows that NotI digestion of E. coli DNA produces five DNA fragments. These fragments can be packaged by the in vitro P1 packaging system of this invention. These fragments are 20 kb, 40 kb, 43 kb (two fragments of this size), and 95 kb in size. After inserting NotI fragments into pNS358 DNA and cloning them in BS591 bacteria as described for Sau3aI inserts, the plasmid DNA from 5 clones was examined. The DNA of two of the cloned plasmids contained only the kan-R domain of pNS358. When the DNA from the other three cloned plasmids were digested with NotI, they were shown to carry NotI fragments greater than 30 kb in size in addition to the kan-R domain of the vector. The size of the NotI inserts in these DNAs was more precisely determined by pulse gel electrophoresis. One of the plasmids (Figure 7B, lane 11) contained a NotI insert that migrated with a 40 kb marker in the gel, while the other two (Figure 7B, lanes 9 and 10) contained inserts that migrate slightly above a 42.5 kb marker. All three clones contained a 12 kb NotI fragment that corresponded to the kan-R domain of the vector. The DNA of one of the clones containing a 43 kb insert (lane 10) also contained a 6 kb NotI fragment, whose origin was unclear as no such E. coli fragment has been described. Digestion of the plasmid DNA shown in lane 10 with BglII and XhoI rather than with NotI produced a family of fragments whose total size exceeds 60 kb, confirming the size estimate based on the NotI digest (Figure 7C, lane 2). Based on their large size, the three plasmids with NotI inserts must have been packaged by the large P1 (P1B) heads.

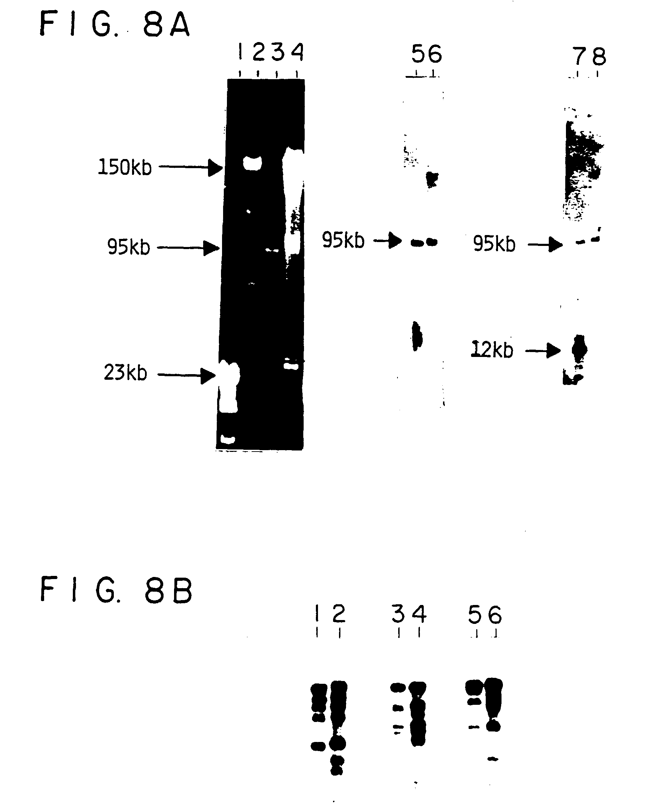

The genes of the E. coli tryptophan biosynthesis operon are present on a 95 kb NotI fragment of E. coli DNA and were utilized to show that a fragment of that size could be packaged into P1 heads in vitro. Phage resulting from the packaging reaction using NotI digested E. coli DNA were used to infect the trp⁻ strain NS3067 of E. coli containing a lambda-P1:cre prophage. The resulting kan-R bacteria were isolated and tested to determine if they could grow on minimal agar in the absence of tryptophan. Two of 50 tested grew. The plasmid DNA was isolated from one of the positive clones and shown by ethidium bromide staining (Figure 8A, lanes 3-4) or by Southern analysis of pulse field gels (Figure 8A, lanes 5-6) to contain a 95 kb E. coli NotI fragment that hybridized to an E. coli trpE gene probe. Lanes 3 and 5 contained the plasmid DNA digested by NotI and lanes 4 and 6 contained E. coli DNA digested with NotI. When the pulse field gel was probed with kan-R gene DNA, a 12 kb DNA fragment appeared in the lane (lane 7) containing the trp plasmid DNA, but not in the lane (lane 8) containing E. coli DNA. That fragment corresponded to the kan-R domain of pNS358 DNA. Digestion of the trp plasmid and E. coli DNA (Figure 8B) with a variety of restriction enzymes (BglII-XhoI, lanes 1 and 2, HindIII, lanes 3 and 4, SmaI, lanes 5 and 6) followed by Southern analysis using the trp plasmid DNA as a probe, indicated that the plasmid DNA contained many fragments that were identical in size to bonafide E. coli DNA fragments. Lanes 1, 3, and 5 contained E. coli DNA, and lanes 2, 4, and 6 contained trp plasmid DNA. Where fragments were present in the E. coli lanes and not in the plasmid lanes, or vice versa, this indicates either that they were derived from the vector or that they represented a junction between vector and E. coli DNA. Taken together, these results indicated that the P1 packaging system can accommodate as much as 107 kb of DNA (the 95 kb NotI insert plus the 12 kb plasmid kan-R domain) and that the vector faithfully replicated that DNA as an extrachromosomal plasmid.

### EXAMPLE 2

### Use of the pNS358-lyt vector to amplify cloned DNA fragments

Two micrograms of pNS358-lyt DNA is digested with BamHI or NotI and used to clone Sau3aI and NotI fragments of E. coli DNA, respectively, as described in Example 1. Following in vitro packaging of the vector DNA with the Sau3aI or NotI fragment inserts as in Example 1, the packaged chimeric DNA is injected into strain NS2974 and kan-R transformants are selected. The DNA of individual kan-R transformants is isolated as described in Example 1 and in the section "Recovery of exogenous DNA fragments cloned into pNS358 DNA following in vitro packaging and transformation of Cre⁺ cells" (pp. 27-28), and is shown to contain E. coli DNA inserts by restriction enzyme digestion and Southern hybridization analysis. To determine if the vector-insert DNA can be amplified by the lytic replicon in the vector, a culture of strain NS2974 bacteria containing a chimeric plasmid is divided in half and grown for 3 hours in either the presence or absence of 1 mM IPTG. At the end of the growth period, total cellular DNA is isolated from each of the two cultures and plasmid copy number measured by Southern hybridization as described for plasmid pNS364 in the section entitled "Amplification of plasmid containing a kan-R gene, a P1 plasmid replicon, and a P1 lytic replicon, pNS364": DNA from the culture grown with IPTG contains 20-30 times more vector insert plasmid DNA than does the DNA isolated from the culture grown without IPTG. This result is confirmed by isolating supercoiled circular DNA from the two cultures using CsCl-ethidium bromide equilibrium density gradients.

## Claims

1. A vector for cloning DNA fragments comprising the following sequence: bacteriophage P1 loxP site-ampicillin resistance gene-pBR322 ori-bacteriophage P1 pac-bacteriophage P1 loxP site-polylinker cloning site-kanamycin resistance gene-bacteriophage P1 plasmid replicon wherein said sequence is circularized such that the bacteriophage P1 plasmid replicon is attached to the first bacteriophage P1 loxP site so that the sequence reads in a counterclockwise direction.

2. A vector according to claim 1 wherein said vector has a bacteriophage P1 lytic replicon under control of a lacZ promoter inserted between the polylinker cloning site and the kanamycin resistance gene.

3. Bacterial strains lysogenized with quadruple phage mutants selected from the group consisting of P1rm⁻cm-2cl.100 9.16 designated NS2961 (ATCC 67665) and P1rm⁻cm-2cl.100 10.1 designated NS2962 (ATCC 67664).

4. Cre⁺ gram-negative bacterial strain having a lacI^{q} repressor designated NS2974 (ATCC 53759).

5. A method of cloning and controlling amplification of DNA fragments as large as 95 kb comprising:
(a) inserting an exogenous DNA fragment into the polylinker cloning site of the vector of claim 2;
(b) contacting the product of step (a) with bacteriophage P1 pac-cleavage proficient extract and head-tail proficient extract;
(c) infecting a Cre⁺ gram-negative bacterial strain having a lacI^{q} repressor with the product of step (b);
(d) derepressing the lacI^{q} repressor by adding IPTG to the product of step (c); and
(e) recovering the cloned and amplified vector DNA.

6. A method according to claim 5 wherein the Cre⁺ gram-negative bacterial strain is the bacterial strain designated NS2974 (ATCC 53759).

7. A method according to claim 5 wherein said bacteriophage P1 pac-cleavage proficient extract is prepared from lysogenic bacterial strain designated NS2962 (ATCC 67664).

8. A method according to claim 5 wherein said head-tail proficient extract is prepared from lysogenic bacterial strain designated NS2961 (ATCC 67665).

9. A process for in vitro P1 bacteriophage packaging of the DNA of the vector of claim 1 or 2 having an exogenous DNA fragment inserted therein comprising contacting the DNA of the fragment-containing vector with bacteriophage P1 pac-cleavage proficient extract and head-tail proficient extract wherein said fragment has a size equal to or less than 95 kb.

10. A process according to claim 9 wherein said bacteriophage P1 pac-cleavage proficient extract is prepared from lysogenic bacterial strain designated NS2962 (ATCC 67664).

11. A process according to claim 9 wherein said head-tail proficient extract is prepared from lysogenic bacterial strain designated NS2961 (ATCC 67665).

12. A method of cloning DNA fragments as large as 95 kb comprising:
(a) inserting an exogenous DNA fragment into the polylinker cloning site of the vector of claim 1;
(b) contacting the product of step (a) with bacteriophage P1 pac-cleavage proficient extract and head-tail proficient extract;
(c) infecting a Cre⁺ gram-negative bacterial strain with the product of step (b); and (d) recovering the cloned vector DNA.

13. A method according to claim 12 wherein the Cre⁺ positive strain is the bacterial strain designated BS591 (ATCC 53760).

## Patentansprüche

1. Vektor zum Klonieren von DNA-Fragmenten, der die folgende Sequenz umfaßt: Bakteriophage-P1-loxP-Stelle - Ampicillin-Resistenz-Gen - pBR322-ori - Bakteriophage-P1-pac - Bakteriophage-P1-loxP-Stelle - Polylinker-Klonierungsstelle - Kanamycin-Resistenz-Gen -Bakteriophage-P1-Plasmid-Replikon, wobei die Sequenz so zirkularisiert ist, daß das Bakteriophage-P1-Plasmid-Replikon an die erste Bakteriophage-P1-loxP-Stelle gebunden ist, so daß die Sequenz im Gegenuhrzeigersinn gelesen wird.

2. Vektor gemäß Anspruch 1, wobei der Vektor ein Bakteriophage-P1-Lyse-Replikon unter der Kontrolle eines *lac*Z-Promotors aufweist, das zwischen der Polylinker-Klonierungsstelle und dem Kanamycin-Resistenz-Gen eingefügt ist.

3. Bakterienstämme, die mit vierfachen Phagenmutanten lysogenisiert sind und aus der Gruppe ausgewählt sind, die aus Pl*rm*⁻*cm*-2cl.100 9.16, der als NS2961 (ATCC 67665) bezeichnet wird, und P1*rm*^{*-*}*cm*-2cl.100 10.1, der als NS2962 (ATCC 67664) bezeichnet wird, besteht.

4. Cre⁺, Gram-negativer Bakterienstamm, der einen *lac*I^{q}-Repressor aufweist und als NS2974 (ATCC 53759) bezeichnet wird.

5. Verfahren zum Klonieren und Steuern der Amplifikation von DNA-Fragmenten von bis zu 95 kb, umfassend:
(a) Einsetzen eines exogenen DNA-Fragments in die Polylinker-Klonierungsstelle des Vektors gemäß Anspruch 2;
(b) In-Kontakt-Bringen des Produkts aus Schritt (a) mit Bakteriophage-P1-pac-spaltungsfähigem Extrakt und -Kopf-Schwanz-fähigem Extrakt;
(c) Infizieren eines Cre⁺, Gram-negativen Bakterienstamms, der einen *lac*I^{q}-Repressor aufweist, mit dem Produkt aus Schritt (b);
(d) Dereprimieren des *lac*I^{q}-Repressors durch Hinzufügen von IPTG zu dem Produkt aus Schritt (c); sowie
(e) Gewinnen der klonierten und amplifizierten Vektor-DNA.

6. Verfahren gemäß Anspruch 5, wobei es sich bei dem Cre⁺, Gram-negativen Bakterienstamm um den Bakterienstamm handelt, der als NS2974 (ATCC 53759) bezeichnet wird.

7. Verfahren gemäß Anspruch 5, wobei der Bakteriophage-P1-*pac*-spaltungsfähige Extrakt aus einem lysogenen Bakterienstamm hergestellt wird, der als NS2962 (ATCC 67664) bezeichnet wird.

8. Verfahren gemäß Anspruch 5, wobei der Kopf-Schwanz-fähige Extrakt aus einem lysogenen Bakterienstamm hergestellt wird, der als NS2961 (ATCC 67665) bezeichnet wird.

9. Verfahren zur in-vitro-Verpackung der DNA des Vektors gemäß Anspruch 1 oder 2, in den ein exogenes DNA-Fragment eingesetzt ist, in Bakteriophage P1, umfassend das In-Kontakt-Bringen der DNA des das Fragment enthaltenden-Vektors mit Bakteriophage-P1-pac-spaltungsfähigem Extrakt und Kopf-Schwanz-fähigem Extrakt, wobei das Fragment eine Größe von kleiner oder gleich 95 kb hat.

10. Verfahren gemäß Anspruch 9, wobei der Bakteriophage-P1-*pac*-spaltungsfähige Extrakt aus einem lysogenen Bakterienstamm hergestellt wird, der als NS2962 (ATCC 67664) bezeichnet wird.

11. Verfahren gemäß Anspruch 9, wobei der Kopf-Schwanz-fähige Extrakt aus einem lysogenen Bakterienstamm hergestellt wird, der als NS2961 (ATCC 67665) bezeichnet wird.

12. Verfahren zum Klonieren von DNA-Fragmenten von bis zu 95 kb, umfassend:
(a) Einsetzen eines exogenen DNA-Fragments in die Polylinker-Klonierungsstelle des Vektors gemäß Anspruch 1;
(b) In-Kontakt-Bringen des Produkts aus Schritt (a) mit Bakteriophage-P1-pac-spaltungsfähigem Extrakt und -Kopf-Schwanz-fähigem Extrakt;
(c) Infizieren eines Cre⁺, Gram-negativen Bakterienstamms mit dem Produkt aus Schritt (b); sowie
(d) Gewinnen der klonierten Vektor-DNA.

13. Verfahren gemäß Anspruch 12, wobei es sich bei dem Cre⁺-positiven Stamm um den Bakterienstamm handelt, der als BS591 (ATCC 53760) bezeichnet wird.

## Revendications

1. Un vecteur pour cloner des fragments d'ADN comprenant la séquence suivante : site *lox*P de bactériophage P1-gène de résistance à l'ampicilline-ori de pBR322-pac de bactériophage P1-site *loxP* de bactériophage P1-lieur multisite de clonage-gène de résistance à la kanamycineréplicon plasmidique de bactériophage P1, ladite séquence étant circularisée de telle manière que le réplicon plasmidique de bactériophage P1 soit attaché au premier site *lox*P de bactériophage P1 de sorte que la séquence se lise en sens inverse des aiguilles d'une montre.

2. Un vecteur selon la revendication 1, dans lequel ledit vecteur comporte un réplicon lytique de bactériophage P1 sous la commande d'un promoteur *lac*Z inséré entre le lieur multisite de clonage et le gène de résistance à la kanamycine.

3. Souches bactériennes rendues lysogènes par des mutants phagiques quadruples choisis dans le groupe formé par P1*rm*^{*-*}*cm*-2cl.100 9.16, désignée par NS2961 (ATCC 67665), et P1*rm*^{*-*}*cm-*2 cl.100 10.1, désignée par NS2962 (ATCC 67664).

4. Souche bactérienne Gram-négative Cre⁺ ayant un répresseur *lac*I^{q}, désignée par NS2974 (ATCC 53759).

5. Une méthode pour cloner et régler l'amplification de fragments d'ADN de taille aussi grande que 95 kb, consistant à :
(a) insérer un fragment d'ADN exogène dans le lieur multisite de clonage du vecteur de la revendication 2 ;
(b) mettre en contact le produit de l'étape (a) avec un extrait compétent de clivage de *pac* de bactériophage P1 et un extrait compétent de tête-queue ;
(c) infecter une souche bactérienne Gram-négative Cre⁺ ayant un répresseur *lac*I^{q} avec le produit de l'étape (b) ;
(d) dérépresser le répresseur *lac*I^{q} par addition d'IPTG au produit de l'étape (c) ; et
(e) recueillir l'ADN du vecteur cloné et amplifié.

6. Une méthode selon la revendication 5, dans laquelle la souche bactérienne Gram-négative Cre⁺ est la souche bactérienne désignée par NS2974 (ATCC 53759).

7. Une méthode selon la revendication 5, dans laquelle ledit extrait compétent de clivage de *pac* de bactériophage P1 est préparé à partir de la souche bactérienne lysogène désignée par NS2962 (ATCC 67664).

8. Une méthode selon la revendication 5, dans laquelle ledit extrait compétent de tête-queue est préparé à partir de la souche bactérienne lysogène désignée par NS2961 (ATCC 67665).

9. Un procédé pour l'encapsidation *in vitro* dans le bactériophage P1 de l'ADN du vecteur de la revendication 1 ou 2 dans lequel est inséré un fragment d'ADN exogène, consistant à mettre en contact l'ADN du vecteur contenant le fragment avec un extrait compétent de clivage de *pac* de bactériophage P1 et un extrait compétent de tête-queue, ledit fragment ayant une taille égale ou inférieure à 95 kb.

10. Un procédé selon la revendication 9, dans lequel ledit extrait compétent de clivage de *pac* de bactériophage P1 est préparé à partir de la souche bactérienne lysogène désignée par NS2962 (ATCC 67664).

11. Un procédé selon la revendication 9, dans lequel ledit extrait compétent de tête-queue est préparé à partir de la souche bactérienne lysogène désignée par NS2961 (ATCC 67665).

12. Une méthode pour cloner des fragments d'ADN de taille aussi grande que 95 kb, consistant à :
(a) insérer un fragment d'ADN exogène dans le lieur multisite de clonage du vecteur de la revendication 1
(b) mettre en contact le produit de l'étape (a) avec un extrait compétent de clivage de *pac* de bactériophage P1 et un extrait compétent de tête-queue ;
(c) infecter une souche bactérienne Gram-négative Cre⁺ avec le produit de l'étape (b) ; et
(d) recueillir l'ADN du vecteur cloné.

13. Une méthode selon la revendication 12, dans laquelle la souche positive Cre⁺ est la souche bactérienne désignée par BS591 (ATCC 53760).
